# EUROPEAN PATENT APPLICATION

(11) **EP 3 006 434 A1**
(43) Date of publication of application: **13.04.2016**
(21) Application number: 14800672.9
(22) Date of filing: 26.05.2014
(51) Int. Cl.: C07D 309/10, A61K 31/351, A61P 3/10, A61P 3/00

(54) **NOVEL COMPOUND DERIVED FROM PLANT OF GENUS QUAMOCLIT AND COMPOSITION CONTAINING SAME AS ACTIVE INGREDIENT FOR PREVENTING OR TREATING DIABETES**

(30) Priority: 24.05.2013 KR 20130059270
(71) Applicant: Korea Research Institute of Bioscience and Biotechnology, Daejeon 305-806 (KR)
(72) Inventor: CHUNG, Bong Hyun, Daejeon 305-806 (KR); LEE, Ui Jin, Daejeon 305-806 (KR); YI, So Yeon, Daejeon 305-806 (KR)
(74) Representative: Gallois, Valérie
(86) International application number: PCT/KR2014/004682
(87) International publication number: WO 2014/189347

(57) **Abstract**

The present invention provides a novel compound isolated from a plant of the genus *Quamoclit* and a method for preparing a novel compound isolated from a plant of the genus *Quamoclit* through chemical synthesis, and relates to a novel compound and a composition containing the novel compound as an active ingredient for preventing or treating diabetes and diabetic complications. The compound derived from a plant of the genus *Quamoclit* and the composition containing the compound as an active ingredient according to the present invention have an excellent effect of promoting insulin secretion, thereby exhibiting efficacies in preventing or treating diabetes and the resulting various complications.

## Description

### [Technical Field]

The present invention relates to a novel compound derived from a plant of the genus *Quamoclit* and a composition for preventing or treating diabetes, comprising the same as an active ingredient and, more particularly, to a novel compound isolated from a plant of the genus *Quamoclit,* which has an excellent effect on the stimulation of insulin secretion, thus exhibiting efficacy in preventing or treating diabetes and various diabetic complications, a compound prepared by chemical synthesis, and a composition for preventing or treating diabetes and diabetic complications, comprising the same as an active ingredient.

### [Background Art]

Diabetes refers to a group of metabolic disorders characterized by chronic hyperglycemia resulting from defects in insulin secretion or action. When abnormally high blood glucose levels are continued for a long period time, various complications occur due to chronic metabolic disorders and the resulting chronic vascular injuries.

Diabetes is a typical adult metabolic disease, from which about 5% of the population in the world are suffering, and causes a huge loss of lives and properties. Most diabetic patients take oral therapeutic agents, but a safe therapeutic agent has not yet been developed. Insulin resistance is known to be the most important cause of diabetes, but the exact mechanism of diabetes is still unknown, and it is known that diabetes is caused by genetic predisposition and environmental factors.

Diabetes is the third leading cause of death in the world, and the number of diabetic patients in 2010 is estimated to be about 250 millions. In Korea, the number of diabetic patients is expected to increase continuously in the future. Non-insulin dependent diabetes mellitus (NIDDM) is the seventh leading cause of death in Korea and accounts for more than 90% of diabetic patients. It is called "adult-type diabetes", because it occurs mainly in persons who are over 40 years old. It is a metabolic disorder which is caused by the insufficient production or inappropriate use of insulin (DeFronzo RA et al., Diabetes Care, 15:318, 1992). Although the cause of NIDDM is not yet clearly known, it is believed that NIDDM is caused by both environmental factors such as westernized diets and lifestyles and genetic factors such as obesity and lack of exercise. For the treatment of NIDDM, dietary therapy and exercise therapy are first attempted, and if the therapeutic effects of such therapies are insufficient, drugs are used, and in many cases, insulin is used. Insulin is administered to patients whose blood glucose levels are not controlled by dietary therapy and oral hypoglycemic agents. However, insulin is a protein, and thus it is inactivated by hydrolysis in the gastrointestinal tract. For this reason, it cannot be administered orally and should be injected intravenously or subcutaneously.

Oral hypoglycemic agents improve the sensitivity of cellular insulin receptor and stimulate the pancreas to promote the secretion of insulin, and thus they are used to control blood glucose levels in NIDDM patients. However, oral therapy for NIDDM can cause hypoglycemia, nausea, vomiting, diarrhea, eruption etc. and particularly serious side effects such as fatal lactic acidosis. In addition, oral hypoglycemic agents, when used for a long time, cause cardiovascular disorders or gastrointestinal and hepatic disorders, and thus the long-term use is not recommended. Due to such disadvantages and side effects, among the currently used therapeutic drugs, there are few drugs that can be applied to all diabetic patients due to their satisfactory efficacy and safety without side effects. Thus, there is an urgent need to develop a more efficient drug for treating diabetes, particularly NIDDM.

About 10 years after the onset of diabetes, almost all the organs of the body are damaged, causing complications. These complications include acute diseases such as hypoglycemia, ketoacidosis, hyperosmolar nonketotic hyperglycemia, hyperglycemic coma, diabetic ketoacidosis, etc. and chronic diseases such as diabetic retinopathy, diabetic cataract, diabetic nephropathy, diabetic neuropathy, cardiovascular complications, viral infections, etc. Chronic diabetic nephropathy is the most important cause of hemodialysis and end-stage renal failure, and diabetic cataract causes blindness, and eventually leads to death.

The mechanisms that cause diabetic complications are generally described by nonenzymatic glycation of protein, polyol pathways, etc. Nonenzymatic glycation of protein indicates a condensation reaction of reducing sugars and amino acid groups such as lysine residue of protein, which is the Maillard reaction, without being mediated by an enzyme. As a result of this reaction, advanced glycation end products are generated. Nonenzymatic glycation of protein can be divided into two steps: (1) wherein amino acid groups such as lysine residue of protein react with aldehydes or ketones of reducing sugars without enzyme activity, which is a nucleophilic addition reaction, to produce the early stage product, Schiff bases, and then ketoamine adducts residing close to the Schiff base react with each other by condensation to produce reversible Amadori-type early glycation products; and (2) wherein when hyperglycemia status continues, the reversible Amadori-type early glycation products are not degraded, but rearranged and cross-linked with proteins, leading to the generation of irreversible advanced glycation end products.

Unlike the reversible Amadori-type early glycation products, the advanced glycation end products are irreversible reaction products, and once generated, they are not degraded even when the blood glucose level is recovered to normal and are accumulated in tissues as long as proteins survive, resulting in abnormal changes in structure and functions of tissues to cause complications all over tissues (Vinson, J. A. et al., 1996, J. Nutritional Biochemistry 7: 559-663; Smith, P. R. et al., 1992, Eur. J. Biochem., 210: 729-739).

For example, glycated albumin, one of the advanced glycation end products produced by the reaction between glucoses and many kinds of proteins, plays a critical role in causing chronic diabetic nephropathy. Glycated albumin can be introduced into glomerular cells more easily than normal albumin, and high concentration of glucose stimulates mesangium cells to increase extracellular matrix synthesis. Due to excessively introduced glycated albumin and increased extracellular matrix, glomerular fibrosis is induced. By such a mechanism, glomerulus is continuously damaged, and at last, there is no choice but to use extreme therapeutic methods such as hemodialysis or organ transplantation. In addition, it has been reported that due to chronic diabetes, collagens are conjugated with the advanced glycation end products in arterial wall and basement membrane proteins are conjugated with the advanced glycation end products in glomerulus, which are accumulated in tissues (Brownlee, M., et al., 1986, Sciences, 232, 1629-1632:1986).

Such nonenzymatic glycation of protein induces glycosylation of basement membrane, proteins such as plasma albumin, lens protein, fibrin, collagen, etc., and the advanced glycation end products generated thereby causes abnormal changes in the structure and functions of tissues, leading to chronic diabetic complications such as diabetic retinopathy, diabetic cataract, diabetic nephropathy, diabetic neuropathy, etc. (Yokozawa, T. et al., J. of Trad. Med., 18: 107, 2001). Thus, it was revealed that it is very important to inhibit the formation of advanced glycation end products in order to postpone, prevent or treat diabetic complications (Brownlee, M. et al., N. Engl. Med. , 318: 1315, 1988).

Accordingly, the present inventors have made extensive efforts to find a natural herbal substance for treating diabetes and diabetic complications with fewer side effects, and as a result, the inventors have obtained a compound with a low molecular weight by enzymatic treatment of a novel compound isolated from a plant of the genus *Quamoclit,* which is disclosed in Korean Patent Application No. 10-2012-0064524, and found that the thus obtained active compound with a low molecular weight further increases insulin secretion in animal cells, thereby completing the present invention.

### [Disclosure]

### [Technical Problem]

It is an object of the present invention to provide a novel compound derived from a natural herbal substance for treating diabetes and diabetic complications with fewer side effects and a method for preparing the same.

Another object of the present invention is to provide an active compound with a low molecular weight obtained by enzymatic treatment of a novel compound derived from a natural herbal substance for treating diabetes and diabetic complications with fewer side effects and a method for preparing the same.

Still another object of the present invention is to provide a pharmaceutical composition or health functional food for preventing or treating diabetes and diabetic complications, comprising the active compound with a low molecular weight as an active ingredient.

Yet another object of the present invention is to provide a compound obtained by chemical synthesis of a novel compound derived from a natural herbal substance for treating diabetes and diabetic complications and a method for preparing the same.

Still yet another object of the present invention is to provide a pharmaceutical composition or health functional food for preventing or treating diabetes and diabetic complications, comprising the compound prepared by chemical synthesis as an active ingredient.

### [Technical Solution]

To achieve the above object, the present invention provides a compound represented by the following chemical formula 1: 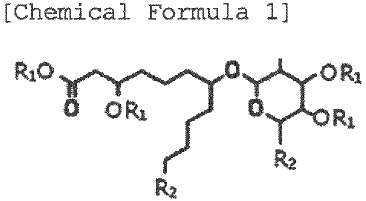 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

The present invention also provides a method for preparing a compound represented by the following chemical formula 1, the method comprising the steps of: (a) extracting a plant of the genus *Quamoclit* with a solvent to obtain a *Quamoclit* extract; (b) fractionating the *Quamoclit* extract with a solvent to obtain a water fraction; and (c) isolating and purifying the water fraction, followed by enzymatic treatment or chemical treatment to obtain the compound represented by chemical formula 1: 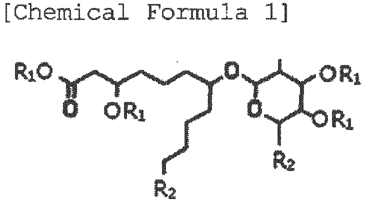 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

Moreover, the present invention provides a method for preparing a compound represented by chemical formula 1, the method comprising the step of enzymatically or chemically treating a compound represented by the following chemical formula 3 to obtain the compound represented by chemical formula 1: 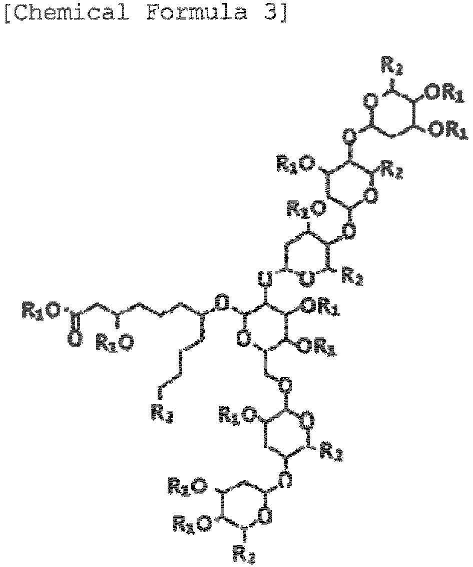 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

Furthermore, the present invention provides a pharmaceutical composition for preventing or treating diabetes or diabetic complications, comprising a compound represented by chemical formula 1; a salt thereof; or an extract comprising the compound represented by chemical formula 1 or a salt thereof as an active ingredient.

In addition, the present invention provides a health functional food for preventing diabetes and diabetic complications, comprising a compound represented by chemical formula 1; a salt thereof; or an extract comprising the compound represented by chemical formula 1 or a salt thereof as an active ingredient.

Moreover, the present invention provides a chemically synthesized compound represented by the following chemical formula 1 and provides a pharmaceutical composition for preventing or treating diabetes or diabetic complications or a health functional food for preventing diabetes or diabetic complications, comprising a compound represented by chemical formula 1; a salt thereof; or a compound comprising the compound represented by chemical formula 1 or a salt thereof as an active ingredient: 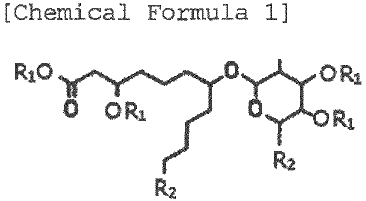 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁-₇ alkoxy, C₁₋₇ haloalkoxy, and hydroxy.

Furthermore, the present invention provides a compound represented by the following chemical formula 6 and provides a pharmaceutical composition for preventing or treating diabetes or diabetic complications or a health functional food for preventing diabetes or diabetic complications, comprising a compound represented by chemical formula 6; a salt thereof; or a compound comprising the compound represented by chemical formula 6 or a salt thereof as an active ingredient: 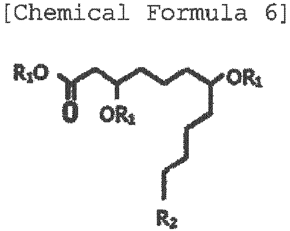 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁-₇ alkoxy, C₁₋₇ haloalkoxy, and hydroxy.

In addition, the present invention provides a method for preventing or treating diabetes and diabetic complications, the method comprising the step of administering a pharmaceutical composition comprising a compound represented by chemical formula 1 or 6; a salt thereof; or an extract comprising the compound represented by chemical formula 1 or 6 or a salt thereof as an active ingredient to a patient in need thereof.

### [Description of Drawings]

FIG. 1 shows the H-NMR spectra, obtained using D₂O as a solvent, of a novel compound derived from *Quamoclit angulata* obtained in accordance with an embodiment of the present invention.
FIG. 2 shows the C-NMR spectra, obtained using D₂O as a solvent, of a novel compound derived from *Quamoclit angulata* obtained in accordance with an embodiment of the present invention.

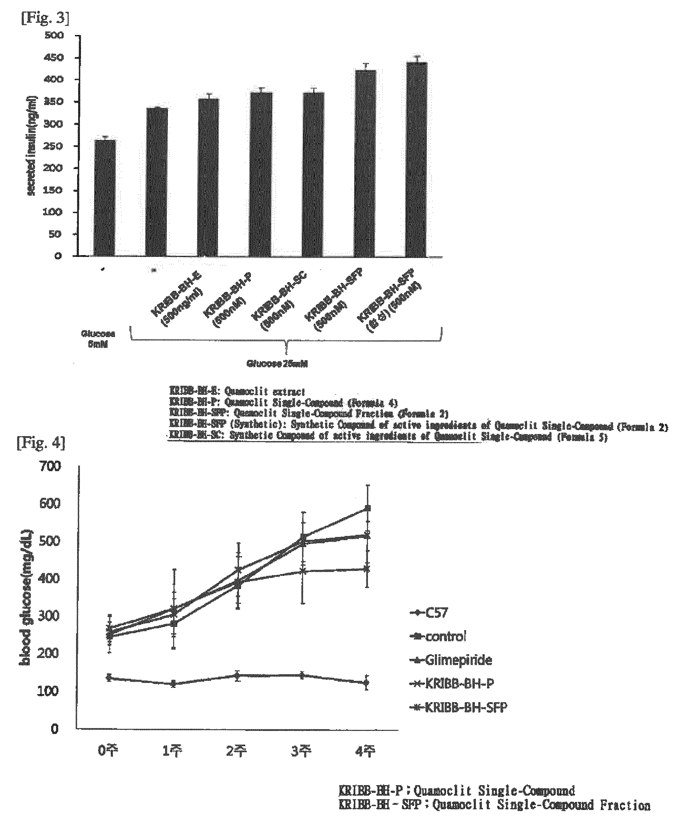

FIG. 3 is a graph showing the stimulation of insulin protein production in a mouse pancreatic beta-cell line by a novel compound derived from *Quamoclit angulata* obtained in accordance with an embodiment of the present invention (KRIBB-BH-P: *Quamoclit-derived* single-compound (chemical formula 4), KRIBB-BH-SFP: *Quamoclit*-derived single-compound fraction (chemical formula 2), KRIBB-BH-SFP (synthetic): *Quamoclit*-derived single-compound fraction using synthetic method (chemical formula 2), KRIBB-BH-SC: *Quamoclit*-derived single-compound fraction using synthetic method (chemical formula 5)).
FIG. 4 shows the effect of a novel compound derived from *Quamoclit angulata* of the present invention on the lowering of blood glucose levels in diabetic animal models.
FIG. 5 shows the effect of a novel compound derived from *Quamoclit angulata* of the present invention on the lowering of blood glucose levels over time according to the results of fasting blood glucose test.
FIG. 6 shows the effect of a novel compound derived from *Quamoclit angulata* of the present invention on the lowering of blood glucose levels according to the results of fasting blood glucose test.

### [Mode for Invention]

Unless otherwise defined, all technical and scientific terms used herein have the same meaning as commonly understood by one of ordinary skill in the art to which the present invention belongs. In general, the definitions of various terms used herein are well known in the art and commonly used.

According to the present invention, it has been tried to obtain a compound with a low molecular weight by enzymatic treatment of a novel compound isolated from a plant of the genus *Quamoclit,* which is disclosed in Korean Patent Application No. 10-2012-0064524, and prove that the thus obtained compound with a low molecular weight further increases insulin secretion in animal cells.

In the present invention, a preparation method has been carried out, comprising the steps of: (a) extracting *Quamoclit* with a solvent selected from the group consisting of water, an organic solvent, and a mixture thereof to obtain a *Quamoclit* extract; (b) suspending the *Quamoclit* extract by addition of water, followed by sequential fractionation with normal hexane, ethyl acetate, and butanol to obtain a water fraction; (c) isolating and purifying the water fraction to obtain a single-compound; and (d) enzymatically or chemically treating the single-compound to obtain a compound represented by chemical formula 1.

As a result, it can be found that the resulting compound of chemical formula 1 increases the production of insulin in a pancreatic beta-cell line.

Accordingly, in one aspect, the present invention is directed to a compound represented by the following chemical formula 1: 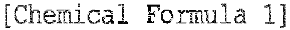 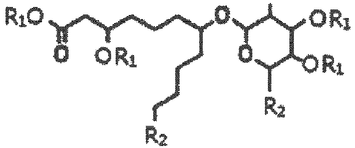 wherein R₁ and R₂ are each independently hydrogen, C₁₋₂₀ alkyl, C₆₋₃₀ aryl, C₂₋₂₀ allyl, or C₆₋₃₀ arylalkyl or acyl.

Preferably, in chemical formula 1, R₁ and R₂ may be each independently H or C₁₋₂₀ alkyl, more preferably, both R₁ and R₂ are H.

The C₁₋₂₀ alkyl may be methyl, ethyl, propyl, or butyl and may be unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

The C₆₋₃₀ aryl may be phenyl, chlorophenyl, or tolyl and may be unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

More preferably, the compound of chemical formula 1 may have a structure represented by the following chemical formula 2:

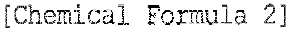

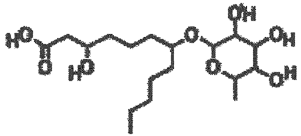

In the present invention, the compound of chemical formula 1 may be isolated from a plant of the genus *Quamoclit,* preferably from *Quamoclit angulata.*

*Quamoclit angulata* that is used in the examples of the present invention is an annual vine plant belonging to the family Convolvulaceae of the order Tubiflorae. It is native to tropical Africa and is cultivated mainly for ornamental purposes. It is characterized in that the vines grow similar to a morning glory and are rolled up on the left side. The whole plant has a length of about 3 m. The leaves are alternate with long petioles and are heart-shaped. The tips of a leaf are acute and the both ends of the lower region in the leaf are pointed. The flowers bloom between August and September and are yellowish red, and 3-5 flowers are suspended from each stalk. The flower resembles the appearance of a morning glory and has 5 sepals, 5 stamens and 1 pistil. The fruit is capsular, ripens in September and has a sepal remaining thereon. This plant is similar to *Quamoclit coccinea,* but the leaf is not divided.

The compound having the structure of chemical formula 2 according to the present invention is derived from a plant of the genus *Quamoclit* and is an active compound with a low molecular weight which is obtained by enzymatic treatment and has an elemental formula of C₁₈H₂₈O₈ and a structure of chemical formula 1. In the present invention, the compound having the structure of chemical formula 2 is named KRIBB-BH-SFP. As a result of MALDI-TOF analysis, the novel compound showed a molecular ion m/Z 378.9, and additionally, NMR assignment was completed through 1D-NMR (H-NMR, C-NMR).

The novel compound according to the present invention can be prepared using a known method. For example, the compound may be extracted with a solvent such as an organic solvent such as water, alcohol, aqueous solution of alcohol, or a mixture thereof. Preferably, the compound may be extracted with water or alcohol and may further be purified with activated carbon.

In another aspect, the present invention is directed to a method for preparing a compound represented by chemical formula 1, the method comprising the steps of: (a) extracting a plant of the genus *Quamoclit* with a solvent to obtain a *Quamoclit* extract; (b) fractionating the *Quamoclit* extract with a solvent to obtain a water fraction; and (c) isolating and purifying the water fraction, followed by enzymatic treatment or chemical treatment to obtain the compound represented by chemical formula 1.

In still another aspect, the present invention is directed to a method for preparing a compound represented by chemical formula 1, the method comprising the steps of: (a) extracting *Quamoclit* with a solvent selected from the group consisting of water, an organic solvent, and a mixture thereof to obtain a *Quamoclit* extract; (b) suspending the *Quamoclit* extract by addition of water, followed by sequential fractionation with normal hexane, ethyl acetate, and butanol to obtain a water fraction; (c) isolating and purifying the water fraction to obtain a compound represented by the following chemical formula 3; and (d) enzymatically or chemically treating the compound represented by chemical formula 3 to obtain the compound represented by chemical formula 1: 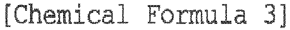 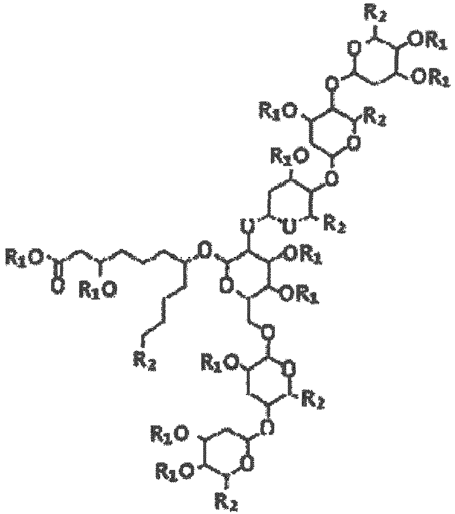 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁-₇ alkoxy, C₁₋₇ haloalkoxy, and hydroxy.

In yet another aspect, the present invention is directed to a method for preparing a compound represented by chemical formula 1, the method comprising the step of enzymatically or chemically treating the compound represented by chemical formula 3 to obtain the compound represented by chemical formula 1.

In step (a), it is preferred that *Quamoclit* is dried and used and that the *Quamoclit* extract is first isolated and purified using a column packed with activated carbon. The isolation and purification in step (c) may be performed by chromatography, for example, but not limited thereto. Chemical treatment may be employed in addition to the enzymatic treatment in step (d).

The enzymatic treatment may comprise the steps of: (i) treatment with at least one enzyme selected from the group consisting of cellulase, beta-glucanase, and xylanase; and (ii) treatment with at least one enzyme selected from the group consisting of glucosidase, cellulase, galactosidase, amyloglucosidase, xylosidase, and xylanase.

The chemical treatment may be performed by addition of an acidic or basic solution in a temperature range of 80 to 120 °C for 1 to 6 hours, preferably for 2 to 4 hours. Preferably, the chemical treatment is performed by addition of 2N HCl or 2N KOH at 100 °C for 3 hours.

In one embodiment of the present invention, the compound of chemical formula 2 is prepared by the following method.

Crushed *Quamoclit angulata* is extracted with a solvent selected from the group consisting of water, alcohol, an organic solvent, and a mixture thereof in an ultrasonic extractor at room temperature for 15 minutes at every 2 hours for 2-3 days or hot-water extracted with water as a solvent at 50 °C. The resulting extract is extracted under reduced pressure in a rotary vacuum evaporator at room temperature, and the extracted residue is dried in a vacuum freeze dryer, thereby obtaining a *Quamoclit angulata* extract dissolved in water.

The obtained *Quamoclit angulata* extract is passed through a column packed with activated carbon to absorb the active ingredients thereof onto the activated carbon, and then, the column packed with activated carbon is washed with distilled water to remove non-adsorbed components. Then, to the column packed with activated carbon from which the non-adsorbed components are removed, an organic solvent such as 10-50% (v/v) ethanol is supplied with increased concentrations such that the active ingredients of *Quamoclit angulata* adsorbed onto the activated carbon are purified by elution. Then, the resulting *Quamoclit angulata* extract is collected. The thus purified *Quamoclit angulata* extract is concentrated under reduced pressure in a rotary vacuum evaporator at room temperature, and the concentrated extract is freeze-dried in vacuum and then dissolved in water to obtain an aqueous solution of *Quamoclit angulata* extract.

The aqueous solution of *Quamoclit angulata* extract is suspended with distilled water and then fractionated with normal hexane (n-hexane), ethyl acetate (Et0Ac), and then butanol (BuOH) to obtain an n-hexane fraction, an Et0Ac fraction, a BuOH fraction, and a water fraction, respectively.

The inhibition of VEGF production is tested using the solvent fractions, respectively, and the water fraction which shows the excellent efficacy is divided into 10 fractions through column chromatography using C18 reversed-phase silica gel as a stationary phase and acetonitrile-water mixed solvent (1:9→9:1 v/v) as a mobile phase, and Sephadex column chromatography (5.0 X 65 cm, MeOH) is performed on the fraction ACN20 [eluted with acetonitrile-DW (2:8 v/v)] to give purifying a novel compound (chemical formula 3).

0.1% Viscozyme (Novozymes Co. Denmark) mainly composed of cellulase, beta-glucanase, and xylanase is added to the obtained novel compound and subjected to enzymatic reaction while stirring at 30 °C for 24 hours. Then, 20 ml of methanol is added to stop the enzymatic reaction, followed by centrifugation. A portion of the supernatant is collected and subjected to high-performance liquid chromatography.

For the removal of sugar, at least one enzyme selected from the group consisting of glucosidase, cellulase, galactosidase, amyloglucosidase, xylosidase, and xylanase is added to the obtained novel compound for further enzymatic reaction. During the enzymatic reaction, the acidity may preferably be in the range of 4.0 to 5.5, and the temperature may preferably be 30 to 50 °C.

In another embodiment of the present invention, it can be found that the treatment of mouse pancreatic cells with KRIBB-BH-SFP, an active compound with a low molecular weight obtained by enzymatic treatment of the novel compound derived from *Quamoclit angulata* of the present invention, stimulates insulin protein production.

Accordingly, in still yet another aspect, the present invention is directed to a pharmaceutical composition or health functional food for preventing or treating diabetes or diabetic complications, comprising a compound represented by chemical formula 1; a salt thereof; or an extract comprising the compound represented by chemical formula 1 or a salt thereof as an active ingredient.

In the present invention, in chemical formula 1, R₁ and R₂ may be each independently H or C₁₋₂₀ alkyl, more preferably, have the structure of chemical formula 2.

The compound having the structure of chemical formula 2 according to the present invention is derived from a plant of the genus *Quamoclit* and is obtained by chemical synthesis of the compound having the structure of chemical formula 1, and in the present invention, the compound having the structure of chemical formula 2 is named KRIBB-BH-SFP (synthetic).

The pharmaceutical composition or health functional food for preventing or treating diabetes or diabetic complications according to the present invention may further comprise the compound represented by chemical formula 1, and the compound represented by chemical formula 1 may preferably be represented by chemical formula 2.

In a further aspect, the present invention is directed to a compound represented by chemical formula 1, and the compound represented by chemical formula 1 may preferably be a compound represented by chemical formula 2.

Moreover, the present invention is directed to a pharmaceutical composition or health functional food for preventing or treating diabetes or diabetic complications, comprising a compound represented by chemical formula 1; a salt thereof; or a compound comprising the compound represented by chemical formula 1 or a salt thereof as an active ingredient.

In another further aspect, the present invention is directed to a method for preparing a compound represented by chemical formula 1, the method comprising the steps of: (a) adding oxalyl chloride and dimethylsulfoxide to 1,5-hexenyl ether for reaction, adding triethylamine to the mixture for reaction, and then further adding alkylmagnesium bromide to the mixture for reaction in a reaction solvent; (b) adding a sugar, acetic anhydride, and iodine to the mixture for reaction, adding iodide to the mixture for reaction, and then further adding sodium hydrogen carbonate and sodium bisulfite to the mixture for reaction in a reaction solvent;
(c) adding a molecular sieve and zinc chloride to the product in step (a) and the product in step (b) for reaction in a reaction solvent; (d) adding osmium tetroxide, sodium periodate, and tetrabutylammonium hydrogen sulfate to the product in step (c) for reaction in a reaction solvent; (e) adding ethylacetate to the product in step (d) for reaction in the presence of lithium diisopropylamide in a reaction solvent; (f) adding sodium alkoxide to the product in step (e) for reaction in a reaction solvent; and (g) adding a base to the product in step (f) for reaction in a reaction solvent.

The sugar in step (b) may comprise at least one selected from the group consisting of glucose, fucose, rhamnose, arabinose, xylose, quinovose, maltose, glucuronic acid, ribose, N-acetyl glucosamine, and galactose, and more preferred is fucose.

The reaction solvent in steps (a) and (e) may be tetrahydrofuran and the reaction solvent in steps (b) and (c) may be dichloromethane.

The reaction solvent in step (d) may be a mixed solution of diethylether and water and the reaction solvent in steps (f) and (g) may be anhydrous methanol.

The alkylmagnesium bromide in step (a) may be pentylmagnesium bromide, and the sodium alkoxide in step (f) may be sodium methoxide.

According to the method for preparing a compound represented by chemical formula 1 according to the present invention, step (a) is carried out in a manner that oxalyl chloride and dimethylsulfoxide are added to 1,5-hexen-1-ol and reacted at a temperature ranging from -60 to -80 °C for 1 to 3 hours, triethylamine is subsequently added to the mixture and reacted at a temperature ranging from -10 to 10 °C for 10 minutes to 3 hours, and then pentylmagnesium bromide is further added to tetrahydrofuran as a solvent and reacted at a temperature ranging from -10 to 10 °C for 10 minutes to 3 hours; step (b) is carried out in a manner that L-(-)-fucose, acetic anhydride, and iodine are added to the mixture and reacted at a temperature ranging from 15 to 35 °C for 1 to 3 hours, aluminum iodide is subsequently added to the mixture and reacted at a temperature ranging from 15 to 35 °C for 1 to 3 hours, and then sodium hydrogen carbonate and sodium bisulfite are further added to the mixture and reacted in dichloromethane as a solvent; step (c) is carried out in a manner that a molecular sieve and zinc chloride are added to the product in step (a) and the product in step (b) and reacted in dichloromethane as a solvent at a temperature ranging from 15 to 35 °C for 5 to 7 hours; step (d) is carried out in a manner that osmium tetroxide, sodium periodate, and tetrabutylammonium hydrogen sulfate are added to the product in step (c) and reacted in a mixed solution of diethylether and water as a solvent at a temperature ranging from 15 to 35 °C for 22 to 26 hours; step (e) is carried out in a manner that ethylacetate is added to the product in step (d) and reacted in anhydrous tetrahydrofuran as a solvent in the presence of lithium diisopropylamide at a temperature ranging from -60 to -80°C for 1 to 3 hours; step (f) is carried out in a manner that sodium alkoxide is added to the product in step (e) and reacted in anhydrous methanol as a solvent at a temperature ranging from -10 to 10 °C for 2 to 4 hours; and step (g) is carried out in a manner that a base is added to the product in step (f) and reacted in anhydrous methanol as a solvent at a temperature ranging from 60 to 80 °C for 30 minutes to 2 hours, thereby preparing a compound represented by chemical formula 2.

In still another embodiment of the present invention, it can be found that the treatment of mouse pancreatic cells with KRIBB-BH-SFP (synthetic) which is obtained by chemical synthesis of KRIBB-BH-SFP, an active compound obtained from a novel compound derived from *Quamoclit angulata,* stimulates insulin protein production.

A compound having a structure of the following chemical formula 5 according to the present invention is derived from a plant of the genus *Quamoclit* and is obtained by chemical synthesis of a compound having a structure of chemical formula 6, and in the present invention, the compound having the structure of chemical formula 5 and obtained by chemical synthesis is named KRIBB-BH-SC):

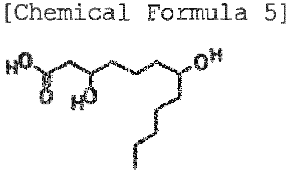

The pharmaceutical composition or health functional food for preventing or treating diabetes or diabetic complications according to the present invention may further comprise the compound represented by chemical formula 6, and the compound represented by chemical formula 6 may preferably be represented by chemical formula 5.

In another further aspect, the present invention is directed to a compound represented by chemical formula 6, and the compound represented by chemical formula 6 may preferably be a compound represented by chemical formula 5.

Moreover, the present invention is directed to a pharmaceutical composition or health functional food for preventing or treating diabetes or diabetic complications, comprising a compound represented by chemical formula 6; a salt thereof; or an extract comprising the compound represented by chemical formula 6 or a salt thereof as an active ingredient.

In still another further aspect, the present invention is directed to a method for preparing a compound represented by chemical formula 6, the method comprising the steps of:
(a) adding oxalyl chloride and dimethylsulfoxide to 1,5-hexenyl ether for reaction, adding triethylamine to the mixture for reaction, and then further adding alkylmagnesium bromide to the mixture for reaction in a reaction solvent;
(b) adding acetic anhydride, pyridine, and 4-dimethylaminopyridine to the product in step (a) for reaction in a reaction solvent; (c) adding osmium tetroxide, sodium periodate, and tetrabutylammonium hydrogen sulfate to the product in step (b) for reaction in a reaction solvent; (d) adding ethylacetate to the product in step (c) for reaction in the presence of lithium diisopropylamide in a reaction solvent; (e) adding sodium alkoxide to the product in step (d) for reaction in a reaction solvent; and (f) adding a base to the product in step (e) for reaction in a reaction solvent.

The reaction solvent in steps (a) and (d) may be tetrahydrofuran and the reaction solvent in steps (b) and (c) may be dichloromethane.

The reaction solvent in step (c) may be a mixed solution of diethylether and water and the reaction solvent in steps (e) and (f) may be anhydrous methanol.

The alkylmagnesium bromide in step (a) may be pentylmagnesium bromide, and the sodium alkoxide in step (e) may be sodium methoxide.

Step (a) is carried out in a manner that oxalyl chloride and dimethylsulfoxide are added to 1,5-hexen-1-ol and reacted at a temperature ranging from -60 to -80 °C for 1 to 3 hours, triethylamine is subsequently added to the mixture and reacted at a temperature ranging from -10 to 10 °C for 10 minutes to 3 hours, and then pentylmagnesium bromide is further added to tetrahydrofuran as a solvent and reacted at a temperature ranging from -10 to 10 °C for 10 minutes to 3 hours; step (b) is carried out in a manner that acetic anhydride, pyridine, and 4-dimethylaminopyridine are added to the product in step (a) and reacted at a temperature ranging from 15 to 35 °C for 1 to 3 hours; step (c) is carried out in a manner that osmium tetroxide, sodium periodate, and tetrabutylammonium hydrogen sulfate to the product in step (b) and reacted in a mixed solution of diethylether and water as a solvent at a temperature ranging from 15 to 35 °C for 22 to 26 hours; step (d) is carried out in a manner that ethylacetate is added to the product in step (c) and reacted in anhydrous tetrahydrofuran as a solvent in the presence of lithium diisopropylamide at a temperature ranging from -60 to -80 °C for 1 to 3 hours; step (e) is carried out in a manner that sodium alkoxide is added to the product in step (d) and reacted in anhydrous methanol as a solvent at a temperature ranging from 15 to 35 °C for 2 to 4 hours; and step (f) is carried out in a manner that a base is added to the product in step (e) and reacted in anhydrous methanol as a solvent at a temperature ranging from 60 to 80 °C for 30 minutes to 2 hours, thereby preparing a compound represented by chemical formula 5.

In yet another embodiment of the present invention, it can be found that the treatment of mouse pancreatic cells with KRIBB-BH-SC, an active compound obtained by chemical synthesis, stimulates insulin protein production.

Examples of the reaction solvents may include aliphatic or aromatic hydrocarbons such as hexane, benzene and toluene, ketones such as acetone and methyl ethyl ketone, esters such as ethylacetate, halogenated hydrocarbons such as dichloromethane, chloroform, and 1,2-dichloromethane, ethers such as dimethyl ether, diisopropyl ether, and tetrahydrofuran, alcohols such as ethanol, propanol, isopropanol, and butanol, N,N-dimethylformamide, N,N-dimethylacetamide, acetonitrile, etc., which can be used alone or in combinations.

Moreover, examples of the reaction bases may include carbonates, hydroxides, hydrides, alkoxides, or alkylated compounds of alkali metals, carbonates, hydroxides, hydrides, alkoxides, or alkylated compounds of alkali earth metals, etc.

In the present invention, the pharmaceutical composition and the health functional food may further comprise an extract obtained by plant-culture or tissue-culture of *Quamoclit.*

The novel compound of the present invention can prevent or treat diabetes and diabetic complications, and examples of the complications may include, but not limited to, hyperglycemia, hyperinsulinemia, hypertriglyceridemia, insulin resistance, dyslipidemia, impaired fasting glucose, impaired glucose tolerance, obesity, arteriosclerosis, microangiopathy, renal disease, heart disease, foot ulcer, arthritis, osteoporosis, and ophthalmologic disease induced by diabetes.

As used herein, the term "diabetes" is intended to include all types of diabetes, including type I diabetes, type 2 diabetes, adult-type diabetes occurring in young people, latent autoimmune diabetes, and pancreatic diabetes, and the diabetic complications include, in addition to the above-mentioned examples, glomerulosclerosis, impotence, diabetic neuropathy, premenstrual syndrome, restenosis, ulcerative colitis, coronary heart disease, hypertension, angina pectoris, myocardial infarction, stroke, skin and connective tissue diseases, metabolic acidosis, symptoms of impaired glucose tolerance, etc.

Moreover, the term "ophthalmologic disease" is intended to include, in addition to diabetic retinopathy, all other ophthalmologic diseases caused by diabetes such as cataract, macular degeneration, external ophthalmoplegia, iridocyclitis, optic neuritis, glaucoma, retinal degeneration, fundus hemorrhage, anomalies of refraction, subconjunctival hemorrhage, and vitreous hemorrhage.

The pharmaceutical composition according to the present invention is essentially composed of a novel compound isolated from *Quamoclit* as an active ingredient and may further comprise at least one pharmaceutically acceptable carrier, excipient, or diluent.

In addition, the pharmaceutical composition of the present invention can also be used in combination with other known agents for treating diabetes or its complications. That is, the pharmaceutical composition of the present invention may be administrated in combination with other known compounds capable of preventing or treating diabetes or its complications.

Accordingly, the pharmaceutical composition for preventing or treating diabetes or diabetic complications of the present invention may further comprise a known antidiabetic compound.

Examples of the antidiabetic compound may include, but not limited to, nateglinide, repaglinide, glitazones, sulfonylureas, metformin, glimepiride, thiazolidinediones, biguanides, α-glucosidase inhibitor such as acarbose, and meglitinides such as prandin.

Meanwhile, in the present invention, the administration route of the pharmaceutical composition may include, but not limited to, oral, intravenous, intramuscular, intra-arterial, intramedullary, intradural, intracardiac, transdermal, subcutaneous, intraperitoneal, intranasal, enteral, local, sublingual, and rectal administrations.

Moreover, for the administration of the pharmaceutical composition, oral and parenteral administrations are preferred. As used herein, the term "parenteral" is intended to include subcutaneous, intradermal, intravenous, intramuscular, intra-articular, intra-bursal, intrasternal, intradural, intralesional, and intracranial injection or implantation techniques.

The pharmaceutical composition of the present invention may be in the form of a sterile injectable preparation such as a sterile injectable aqueous or oleaginous suspension. This suspension may be formulated according to the known art using those suitable dispersing or wetting agents (e.g., Twin 80) and suspending agents. The sterile injectable preparation may also be a sterile injectable solution or suspension in a non-toxic parenterally-acceptable diluent or solvent (e.g., a solution in 1,3-butanediol). Acceptable vehicles and solvents may include mannitol, water, Ringer's solution, or isotonic sodium chloride solution. In addition, sterile, fixed oils are conventionally employed as a solvent or suspending medium. For this purpose, any bland fixed oil may be employed including synthetic mono- or diglycerides. Fatty acids such as oleic acid and glyceride derivatives thereof are useful in the preparation of injectable preparations, like the pharmaceutically acceptable natural oils (e.g., olive oil or castor oil), and particularly, polyoxyethylated derivatives thereof.

The pharmaceutical compositions of the present invention may be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavoring and/or coloring agents may be added.

The pharmaceutical compositions of the present invention may also be administered in the form of suppositories for rectal administration. These compositions can be prepared by mixing a compound of the present invention with a suitable non-irritating excipient which is solid at room temperature but liquid at the rectal temperature. Such materials include, but not limited to, cocoa butter, beeswax and polyethylene glycols.

Topical administration of the pharmaceutical compositions according to the invention is especially useful when the desired treatment involves areas or organs readily accessible by topical application. For topical application topically to the skin, the pharmaceutical composition should be formulated with a suitable ointment containing the active components suspended or dissolved in a carrier. Carriers for topical administration of the compounds of the present invention include, but not limited to, mineral oil, liquid petroleum, white petroleum, propylene glycol, polyoxyethylene polyoxypropylene compound, emulsifying wax, and water. Alternatively, the pharmaceutical composition can be formulated with a suitable lotion or cream containing the active compound suspended or dissolved in a carrier. Suitable carriers include, but not limited to, mineral oil, sorbitan monostearate, polysorbate 60, cetyl esters wax, cetearyl alcohol, 2-octyldodecanol, benzyl alcohol, and water. The pharmaceutical compositions of the present invention may also be topically applied to the lower intestinal tract by rectal suppository formulation or in a suitable enema formulation. Topically-transdermal patches are also included in the present invention.

The pharmaceutical compositions of the present invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

In the pharmaceutical composition of the present invention, the novel compound of the present invention is present in a therapeutically or preventively effective amount effective amount.

However, it will be understood that effective doses for specific patients will also vary depending on various factors including the activity of specific compound used, the patient's age, weight, general health condition, sex, and diet, the administration interval, the administration route, the discharge rate, the possibility of co-usage with other therapeutic treatments, and the severity of disease to be prevented or treated. The pharmaceutical composition of the present invention may be formulated into pills, dragees, capsules, liquids, gels, syrups, suspensions, etc.

In case of administration of the pharmaceutical composition of the present invention into subcutaneous cells of fish, the pharmaceutical composition may be administrated into the gill pouch or digestive tract. The pharmaceutical composition of the present invention may be injected into muscle cells or other cells in muscle tissue and may be injected into visceral cells in the abdominal cavity.

Preferably, the pharmaceutical composition for oral administration may be prepared by mixing the active ingredient with solid excipients and may be prepared in the form of granules to obtain tablets or dragees. Suitable excipients are fillers such as sugars, including lactose, sucrose, mannitol, and sorbitol; celluloses such as maize starch, wheat starch, rice starch, potato starch, gelatin, gum tragacanth, methyl cellulose, hydroxypropylmethylcellulose, sodium carboxymethylcellulose. If desired, disintegrating or solubilizing agents may be added, such as the cross-linked polyvinylpyrrolidone, agar, or alginic acid, or a salt thereof such as sodium alginate.

In a preferred embodiment, for parental administration, the pharmaceutical composition may be formulated into aqueous solutions. Preferably, physiologically compatible buffers such as Hank's solution, Ringer's solution, physiological salt buffer can be used. Aqueous injection suspension may contain substances, which increase the viscosity of the suspension, such as sodium carboxymethyl cellulose, sorbitol or dextran. In addition, suspensions of the active ingredients as appropriate oily injection suspensions may also be administered. Suitable lipophilic solvents or vehicles include fatty oils, such as sesame oil, or synthetic fatty acid esters, such as ethyl oleate, triglycerides, or liposomes. Non-lipid polycationic amino polymers may also be used for delivery. Optionally, the suspension may also contain suitable stabilizers or agents to increase the solubility of the compounds and allow for the preparation of highly concentrated solutions.

Meanwhile, the novel compound derived from *Quamoclit* of the present invention may be prepared in the form of food for preventing or improving diabetes and its complications.

The health functional food of the present invention is a food composition, and examples thereof include all types of food including functional food, nutritional supplement, health food, and food additives. The above types of food may be prepared in various forms by conventional methods known in the art. For example, for the health food, the novel compound derived from *Quamoclit* of the present invention may be prepared in the form of tea, juice and drink, or it may be granulized, encapsulated or powdered. Also, the functional food may be prepared by adding the novel compound derived from *Quamoclit* of the present invention to beverages (including alcoholic beverage), fruits and processed foods thereof (e.g., canned fruit, bottled food, jam, marmalade, etc.), fishes, meats and processed foods thereof (e.g., ham, sausage, corn beef, etc.), bread and noodles (e.g., Japanese noodle, buckwheat noodles, ramen, spaghetti, macaroni, etc.), fruit juice, various drinks, cookies, taffy, milk products (e.g., butter, cheese, etc.), edible plant oil and fat, margarine, vegetable proteins, a retort food, frozen food, various seasonings (e.g., soybean paste, soy sauce, sauces, etc.) and the like.

Moreover, all optically active stereoisomers, diastereomers and racemates of the compound represented by chemical formula 1 and the compound represented by chemical formula 6 according to the present invention are included within the scope of the present invention. That is, the present invention may include R-isomers or S-isomers as their optically active stereoisomers.

The preparation of optically-active forms (for example, by resolution of the racemic form by recrystallization techniques, by chiral synthesis, by enzymatic resolution, by biotransformation, or by chromatographic separation) and the determination of antibacterial activity are well known in the art.

Furthermore, the present invention provides a method for preventing or treating diabetes and diabetic complications, the method comprising the step of administering a pharmaceutical composition comprising a compound represented by chemical formula 1 or 6; a salt thereof; or an extract comprising the compound represented by chemical formula 1 or 6 or a salt thereof as an active ingredient to a patient in need thereof.

### EXAMPLES

Hereinafter, the present invention will be described in further detail with reference to examples. It will be obvious to a person having ordinary skill in the art that these examples are illustrative purposes only and are not to be construed to limit the scope of the present invention.

### Example 1: Isolation of Novel Compound from Quamoclit

51 g of *Quamoclit angulata* collected from Seogwang-ri, Andeok-myeon, Seogwipo-si, Jeju-do, Korea was hot-water extracted with water as a solvent at 50 °C. The resulting extract was concentrated under reduced pressure in a rotary vacuum evaporator at room temperature, and the extracted residue was dried in a vacuum freeze dryer to give a dried *Quamoclit angulata* extract. The concentrate was suspended in water and passed through column chromatography packed with activated carbon to absorb active ingredients thereof onto the activated carbon, and then, the column packed with activated carbon was washed with distilled water to remove non-adsorbed components. Then, to the column packed with activated carbon from which the non-adsorbed components were removed, an organic solvent such as 10-50% (v/v) ethanol is supplied with increased concentrations such that the active ingredients of *Quamoclit angulata* adsorbed onto the activated carbon were purified by elution. Then, the resulting *Quamoclit angulata* extract was collected. The thus purified *Quamoclit angulata* extract was concentrated under reduced pressure, and 5 g of the concentrated *Quamoclit angulata* extract was dissolved in water until the concentration of the *Quamoclit angulata* extract was 20 mg/ml.

The water extract was suspended in distilled water, followed by sequential solvent fractionation with normal hexane (n-hexane), ethyl acetate (Et0Ac), and butanol (BuOH) to give an n-hexane fraction, an Et0Ac fraction, a BuOH fraction, and a water fraction, respectively.

The inhibition of VEGF production was tested using the solvent fractions, respectively, and the water fraction which showed the excellent efficacy was divided into 10 fractions through column chromatography using C18 reversed-phase silica gel as a stationary phase and acetonitrile-water mixed solvent (1:9→9:1 v/v) as a mobile phase, and Sephadex column chromatography (5.0 X 65 cm, MeOH was performed on the fraction ACN20 [eluted with acetonitrile-DW (2:8 v/v)] to give a compound of the following chemical formula 4:

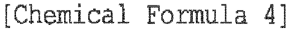

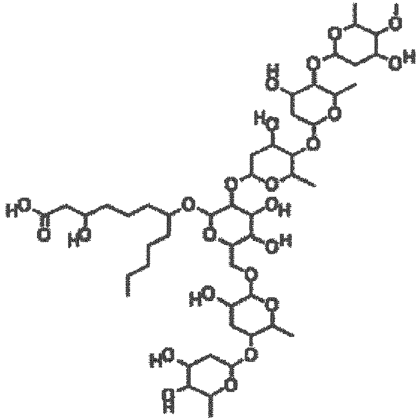

The Conditions used in the experiment and the results are as follows:

### High-Performance Liquid Chromatography (HPLC) Analysis (Shimadzu Model)

Column: YMC ODS-18(4.6 x 250 mm, 4 µm)
Mobile Solvent: 20% Acetonitrile added with 0.1% trifluoroacetic acid
Flow Rate: 1 ml/min

### Example 2: Enzymatic Treatment of Quamoclit-Derived Compound

0.1% Viscozyme (Novozymes Co. Denmark) mainly composed of cellulase, beta-glucanase, and xylanase was added to the *Quamoclit*-derived compound isolated in Example 1 and subjected to enzymatic reaction while stirring at 30 °C for 24 hours. Then, 20 ml of methanol was added to stop the enzymatic reaction, followed by centrifugation. A portion of the supernatant was collected and subjected to high-performance liquid chromatography.

As enzymes for the removal of sugar, 0.1% beta-glucanase and xylanase were added to the obtained novel compound for further enzymatic reaction while stirring at an acidity of 5.5 and at 50 °C for 24 hours. Then, the resulting mixture was heated in hot water for 5 to 15 minutes, and the reaction was stopped. The reaction solution was concentrated under reduced pressure, and the obtained product was subjected to high-performance liquid chromatography, yielding a compound with a low molecular weight.

### High-Performance Liquid Chromatography (HPLC) Analysis (Shimadzu Model)

Column: YMC ODS-18(4.6 x 250 mm, 4 µm)
Mobile Solvent: 30% Acetonitrile added with 0.01% trifluoroacetic acid
Flow Rate: 1 ml/min

### Example 3: Characterization of Quamaclit-Derived Compound

As a result of MALDI-TOF analysis, the compound with a low molecular weight obtained in Example 2 showed a molecular ion m/Z 378.9 and had an elemental formula of C₁₈H₂₈O₈, and additionally, NMR assignment was completed through 1D-NMR (H-NMR, C-NMR).

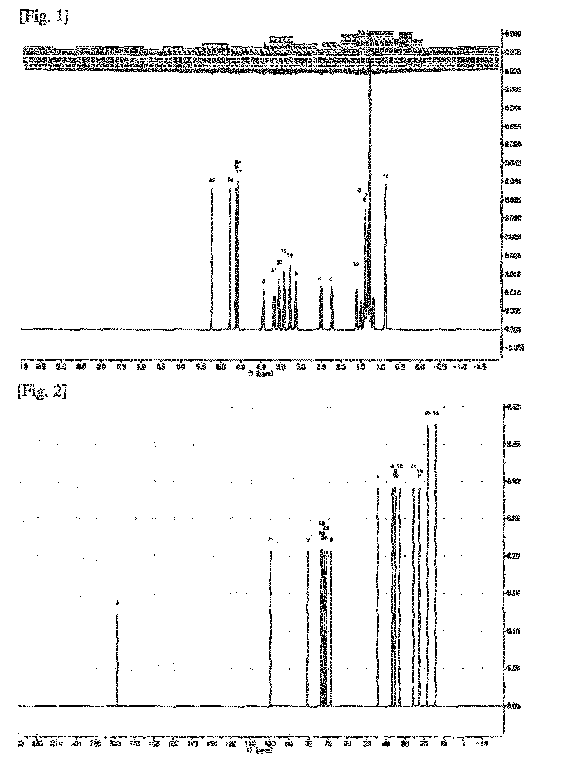

The proton nuclear magnetic resonance (H-NMR) spectra obtained using deuterium-substituted water (D₂O) as a solvent and tetramethylsilane (TMS) as a standard material are shown in FIG. 1. The carbon nuclear magnetic resonance (C-NMR) spectra obtained using deuterium-substituted water (D₂O) as a solvent and tetramethylsilane (TMS) as a standard material are shown in FIG. 2.

It was found from the above results that the active compound with a low molecular weight obtained in Example 2 by enzymatic treatment of the *Quamoclit angulata*-derived compound has an elemental formula of C₁₈H₂₈O₈ and a structure of the following chemical formula 2:

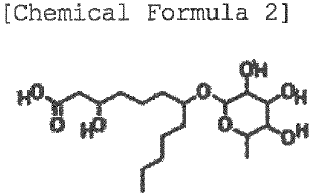

The above compound is named KRIBB-BH-SFP.

### Example 4: Synthesis of Quamoclit-Derived Compound

### Example 4-1: Synthesis of Quamoclit-Derived Compound. (Chemical Formula 2)

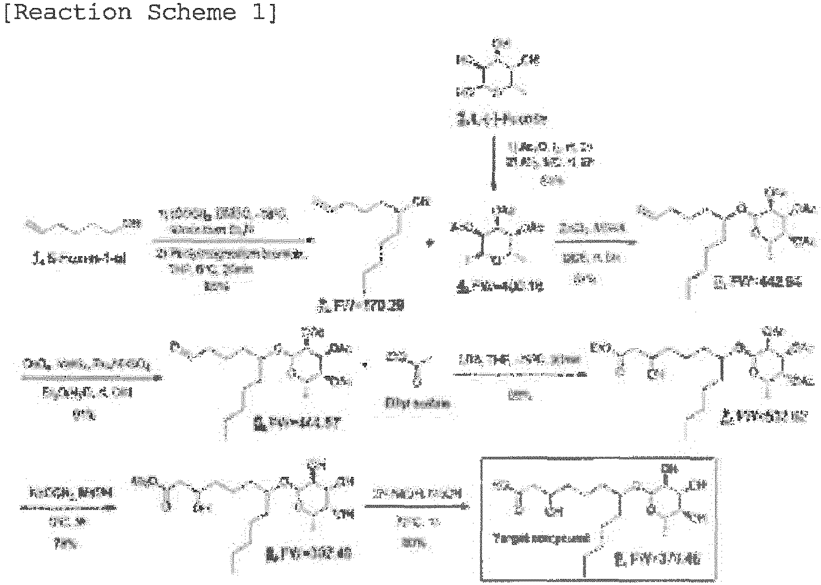

### Example 4-1-1: Synthesis of Compound 2

To 300 mL of dichloromethane cooled to -78 °C, dimethylsulfoxide (4.25 mL, 60.0 mmol) and oxalyl chloride (3.86 mL, 45.0 mmol) were added. Then, 5-hexen-1-ol (3.0 g, 30.0 mmol) was slowly added thereto. Upon completion of the addition, the mixture was stirred at -78 °C for 1.5 hours, slowly added with triethylamine (16.7 mL, 120.0 mmol), and then stirred at 0 °C for 30 minutes. 50 mL of saturated NH₄Cl solution and 50 mL of water were added and extracted with diethylether. 5 g of MgSO₄ was added to the extraction solution, MgSO₄ was removed by filtration, and then the solvent was removed in an evaporator to give a crude product. To the resulting crude product, 300 mL of anhydrous THF was added. To the reaction mixture cooled to 0 °C, pentylmagnesium bromide (22.5 mL, 45.0 mmol, 2.0 M in ether) was added and stirred at 0 °C for 30 minutes. 50 mL of saturated NH₄Cl solution and 50 mL of water were added and extracted with diethylether. 5 g of MgSO₄ was added to the extraction solution, MgSO₄ was removed by filtration, and then the solvent was removed in an evaporator. The resulting crude product was purified by silica gel column chromatography to give 4.36 g of pure compound 2 as a colorless oil (85% in two steps).

### Example 4-1-2: Synthesis of Compound 4

L-(-)-fucose (1.0 g, 6.1 mmol) was added to a round bottom flask (RBF). Acetic anhydride (2.36 mL, 23.01 mmol) and iodine (15.48 mg, 0.061 mmol) were added and stirred at room temperature for 2 hours. Subsequently, 60 mL of dichloromethane was added, and then aluminum iodide (2.98 g, 7.32 mmol) was slowly added. The resulting mixture was stirred at room temperature for 2 hours, mixed with 60 mL of NaHCO₃ aqueous solution and 5 mL of NaHSO₃ aqueous solution, and then extracted with 200 mL of dichloromethane. 5 g of MgSO₄ was added to the extraction solution, MgSO₄ was removed by filtration, and then the solvent was removed in an evaporator. The resulting crude product was purified by silica gel column chromatography to give 2.08 g of pure compound 4 as a. colorless sticky oil (85%).

### Example 4-1-3: Synthesis of Compound 5

To a round bottom flask (RBF) containing 30 mL of dichloromethane, compound 2 (638.5 mg, 3.75 mmol) obtained in Example 4-1-1 and compound 4 (1.0 g, 2.5 mmol) obtained in Example 4-1-2 were added. Subsequently, 5 g of MS4A (Molecular Sieve 4 Å) and ZnCl₂ (375.0 mg, 2.75 mmol) were added and stirred at room temperature for 6 hours. Then, MS4A was removed by filtration, 100 mL of diethylether was added, and washed with 30 mL of saturated NH₄Cl solution and then with 30 mL of brine. 3 g of MgSO₄ was added to the extraction solution, MgSO₄ was removed by filtration, and then the solvent was removed in an evaporator. The resulting crude product was purified by silica gel column chromatography to give 961.0 mg of pure compound 5 (diastereomer mixture) as a colorless oil (87%).

### Example 4-1-4: Synthesis of Compound 6

To a round bottom flask (RBF) containing a mixed solvent of 40 mL of diethylether and 40 mL of water, compound 5 (3.0 g, 6.78 mmol) obtained in Example 4-1-3 was added. Subsequently, OsO₄ (60.3 mg, 0.237 mmol), NaIO₄ (8.48 g, 39.6 mmol), and Bu₄NHSO₄ (80.5 mg, 0.237 mmol) were added and stirred vigorously at room temperature for 24 hours. Then, 200 mL of diethylether was added and washed with 100 mL of water and then with 100 mL of brine. 5 g of MgSO₄ was added to the extraction solution, MgSO₄ was removed by filtration, and then the solvent was removed in an evaporator. The resulting crude product was purified by silica gel column chromatography to give 3.7 g of pure compound 6 as a colorless oil (61%),

### Example 4-1-5: Synthesis of Compound 7

To a round bottom flask (RBF) containing 15 mL of anhydrous THF, ethyl acetate (134.2 mg, 1.524 mmol) was added and cooled to -78 °C. Subsequently, lithium diisopropylamide (LDA, 0.89 mL, 1.78 mmol, 2.0 M in THF/heptane/ethylbenzene) was slowly added and stirred at - 78 °C for 30 minutes. Then, compound 6 (569.0 mg, 1.27 mmol) obtained in Example 4-1-4 was diluted with 5 mL of anhydrous THF and slowly added. The resulting mixture was stirred at -78 °C for 30 minutes, 80 mL of diethylether was added and washed with 20 mL of saturated NH₄Cl solution and then with 20 mL of brine. 3 g of MgSO₄ was added to the extraction solution, MgSO₄ was removed by filtration, and then the solvent was removed in an evaporator. The resulting crude product was purified by silica gel column chromatography to give 600.0 mg of pure compound 7 as a colorless oil (88%).

### Example 4-1-6: Synthesis of Compound 8

To a round bottom flask (RBF) containing 20 mL of anhydrous methanol, compound 7 (960.0 mg, 1.80 mmol) obtained in Example 4-1-5 was added and cooled to 0 °C. Subsequently, sodium methoxide (437.5 mg, 8.10 mmol) diluted with 5 mL of anhydrous methanol was added, stirred at 0 °C for 3 hours, and then the reaction solvent was removed in an evaporator. The resulting crude product was purified by silica gel column chromatography to give 550.0 mg of pure compound 8 as a colorless oil (78%).

### Example 4-1-7: Synthesis of Compound 9

To a round bottom flask (RBF) containing 20 mL of anhydrous methanol, compound 8 (860.0 mg, 2.19 mmol) obtained in Example 4-1-6 was added and cooled to 0 °C. Subsequently, 2N-NaOH (1.31 mL, 2.62 mmol) was slowly added, stirred at 70 °C for 1 hour, and then the reaction solvent was removed in an evaporator. The resulting crude product was purified by silica gel column chromatography to give 665.0 mg of pure compound 9 (chemical formula 2) as a white sticky solid (80%), which has a structure of the following chemical formula 2 and is named KRIBB-BH-SFP (synthetic):

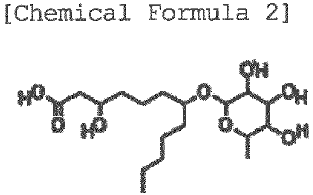

### Example 4-2 Synthesis of Quamoclit-Derived Compound (Chemical Formula 5 )

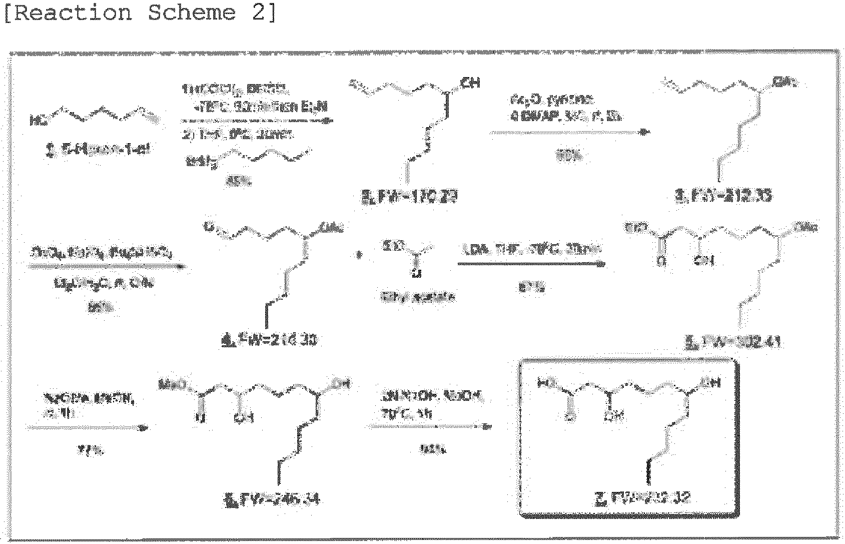

### Example 4-2-1: Synthesis of Compound 2

To 300 mL of dichloromethane cooled to -78 °C, dimethylsulfoxide (4.25 mL, 60.0 mmol) and oxalyl chloride (3.86 mL, 45.0 mmol) were added. Then, 5-hexen-1-ol (3.0 g, 30.0 mmol) was slowly added thereto. Upon completion of the addition, the mixture was stirred at -78 °C for 1.5 hours, slowly added with triethylamine (16.7 mL, 120.0 mmol), and then stirred at 0 °C for 30 minutes. 50 mL of saturated NH₄Cl solution and 50 mL of water were added and extracted with diethylether. 5 g of MgSO₄ was added to the extraction solution, MgSO₄ was removed by filtration, and then the solvent was removed in an evaporator to give a crude product. To the resulting crude product, 300 mL of anhydrous THF was added. To the reaction mixture cooled to 0 °C, pentylmagnesium bromide (22.5 mL, 45.0 mmol, 2.0 M in ether) was added and stirred at 0 °C for 30 minutes. 50 mL of saturated NH₄Cl solution and 50 mL of water were added and extracted with diethylether. 5 g of MgSO₄ was added to the extraction solution, MgSO₄ was removed by filtration, and then the solvent was removed in an evaporator. The resulting crude product was purified by silica gel column chromatography to give 4.36 g of pure compound 2 as a colorless oil (85% in two steps).

### Example 4-2-2: Synthesis of Compound 3

100 mL of dichloromethane was added to compound 2 (2.7 g, 15.9 mmol) obtained in Example 4-2-1, and then pyridine (2.57 mL, 31.8 mmol) and 4-dimethylaminopyridine (4-DMAP, 388.5 mg, 3.18 mmol) were added at room temperature. The reaction mixture was cooled to 0 °C, and then acetic anhydride (2.25 mL, 23.85 mmol) was slowly added and stirred at room temperature for 2 hours. 200 mL of diethylether was added and washed with 50 mL of saturated water, with 50 mL of 1N-HCl, and then with 20 mL of brine. Then, 5 g of MgSO₄ was added to the extraction solution, MgSO₄ was removed by filtration, and then the solvent was removed in an evaporator. The resulting crude product was purified by silica gel column chromatography to give 3.2 of pure compound 3 as a colorless oil (95%).

### Example 4-2-3: Synthesis of Compound 4

A mixed solvent of 80 mL of diethylether and 80 mL of water was added to compound 3 (3.78 g, 17.8 mmol) obtained in Example 4-2-2. Subsequently, OsO₄ (35.3 mg, 0.623 mmol), NaIO₄ (22.4 g, 106.8 mmol), and Bu₄NHSO₄ (148.5 mg, 0.623 mmol) were added and stirred vigorously at room temperature for 24 hours. Then, 200 mL of diethylether was added and washed with 100 mL of water and then with 50 mL of brine. 5 g of MgSO₄ was added to the extraction solution, MgSO₄ was removed by filtration, and then the solvent was removed in an evaporator. The resulting crude product was purified by silica gel column chromatography to give 2.51 g of pure compound 4 as a colorless oil (66%).

### Example 4-2-4: Synthesis of Compound 5

To a round bottom flask (RBF) containing 6 mL of anhydrous THF, ethyl acetate (53.8 mg, 0.66 mmol) was added and cooled to -78 °C. Subsequently, lithium diisopropylamide (LDA, 0.36 mL, 0.72 mmol, 2.0 M in THF/heptane/ethylbenzene) was slowly added and stirred at - 78 °C for 30 minutes. Then, compound 4 (130.0 mg, 0.60 mmol) obtained in Example 4-2-3 was diluted with 5 mL of anhydrous THF and slowly added. The resulting mixture was stirred at -78 °C for 30 minutes, 50 mL of diethylether was added and washed with 10 mL of saturated NH₄Cl solution and then with 10 mL of brine. 1 g of MgSO₉ was added to the extraction solution, MgSO₄ was removed by filtration, and then the solvent was removed in an evaporator. The resulting crude product was purified by silica gel column chromatography to give 157.8 mg of pure compound 5 as a colorless oil (87%).

### Example 4-2-5: Synthesis of Compound 6

6 mL of anhydrous methanol was added to compound 5 (190.0 mg, 0.63 mmol) obtained in Example 4-2-4 and then cooled to 0 °C. Subsequently, sodium methoxide (102.0 mg, 1.89 mmol) diluted with 2 mL of anhydrous methanol was added, stirred at room temperature for 1 hour, and then the reaction solvent was removed in an evaporator. The resulting crude product was purified by silica gel column chromatography to give 120.0 mg of pure compound 6 as a sticky solid (77%).

### Example 4-2-6: Synthesis of Compound 7

5 mL of anhydrous methanol was added to compound 6 (120.0 mg, 0.48 mmol) obtained in Example 4-2-5 and then cooled to 0 °C. Subsequently, 2N-NaOH (0.48 mL, 0.97 mmol) was slowly added, stirred at 70 °C for 1 hour, and then the reaction solvent was removed in an evaporator. The resulting crude product was purified by silica gel column chromatography to give 105.0 mg of pure compound 7 (chemical formula 5) as a white sticky solid (93%), which has a structure of the following chemical formula 5 and is named KRIBB-BH-SC :

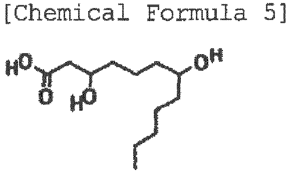

### Example 5: Determination of Effect of Quamoclit-Derived Compound on Stimulation of Insulin Secretion

The effect of the novel compound KRIBB-BH-SFP isolated from *Quamoclit angulata* on the stimulation of insulin secretion was determined in a mouse pancreatic beta-cell line, Min6 cell line.

Min6 cells (ATCC, USA) were cultured in HDMEM supplemented with 15% fetal bovine serum. To induce the expression of insulin in the Min6 cell line, the cells were plated on a 96 well plate at a density of 1 x 10⁴ cell/plate and cultured in serum-free DMEM (high glucose) supplemented with 15% FBS for 72 hours. Then, the cells were washed with PBS buffer solution and cultured in HBSS buffer solution for 2 hours, and then, the buffer solution was replaced with HBSS buffer solution supplemented with 25 mM glucose. Then, the novel compound KRIBB-BH-P was added to a final concentration of 0.5 ng/ml, and the cells were cultured for 2 hours to determine the stimulation of insulin expression. Meanwhile, for use as a control, cells were treated with 5.5 mM glucose such that the insulin was not expressed. For use as a negative control, cells were treated with 25 mM glucose, and the level of insulin expression in these cells was compared to determine the effect of the novel compound (KRIBB-BH-SFP) derived from *Quamoclit angulata* on the stimulation of insulin expression. Culture fluid of Min6 cells cultured by the above-described method was collected to measure the amount of insulin secreted from Min6 cells using Insulin ELISA kit (R&D, UK).

As a result, as shown in FIG. 3, it could be found that the level of insulin expression was increased in the pancreatic beta-cell line treated with the novel compounds KRIBB-BH-SFP, KRIBB-BH-SFP (synthetic), and KRIBB-BH-SC.

### Example 6: Analysis of Diabetes Indicators in Diabetic Mouse Treated with Quamoclit-Derived Novel Compound

### 6-1: Analysis of Blood glucose

Normal mice and diabetic mice were treated with the *Quamoclit*-derived novel compound to determine the changes in diabetes indicators such as blood glucose and glycated hemoglobin.

6-week-old mice (Male C57BL/6J mouse, 20 g, Central Lab. Animal Inc., Seoul) and 6-week-old diabetic mice (Male C57BL/Ks DB/DB mouse, 20 g, Central Lab. Animal Inc., Seoul) were purchased and acclimated under constant temperature (25 °C) and humidity (50%) for 1 week and used for the experiment.

Mice were classified into a normal mouse control group, a diabetic mouse control group, a diabetic mouse group treated with drug (glimepiride), a diabetic mouse group treated with the *Quamoclit angulata*-derived novel compound (KRIBB-BH-P, 1 mg/kg), and a diabetic mouse group treated with the *Quamoclit angulata*-derived novel compound (KRIBB-BH-SFP, 1 mg/kg), each group consisting of five mice. The treated mice were orally administered with the drug and compounds daily for 4 weeks. The change in blood glucose in mice of each group was measured every week, and after 4 weeks, blood was collected from each mouse to measure the concentration of glycated hemoglobin.

As a result, with respect to the change in blood glucose, as shown in FIG. 4, the level of blood glucose was increased in the diabetic mouse control group as compared to the normal mouse control group; however, in the diabetic mouse group treated with the *Quamoclit angulata*-derived novel compound (KRIBB-BH-P, 1 mg/kg) and the diabetic mouse group treated with the *Quamoclit angulata*-derived novel compound (KRIBB-BH-SFP, 1 mg/kg), the level of blood glucose was significantly reduced.

### 6-2: Analysis of Glycated Hemoglobin (HbA1c)

In order to reduce the complications developed in diabetic patients under treatment, it is important to maintain the level of blood glucose at an appropriate level. Since the levels of blood glucose measured at a certain time may be changed by various factors, the test of glycated hemoglobin (HbAlc) is most widely used for the purpose of determining the long-term change in blood glucose control. Glycated hemoglobin is formed when hemoglobin that is normally present in erythrocytes is combined with glucose, and when the blood glucose is maintained at a high level, the level glycated hemoglobin also increases. The level of glycated hemoglobin indicates the average level of blood glucose for 2 to 4 months, and thus it is useful for determining the long-term blood glucose control.

In order to determine the effect of the *Quamoclit angulata*-derived novel compound on the glycated hemoglobin, blood was collected from each mouse in the normal mouse control group, the diabetic mouse control group, the diabetic mouse group treated with the *Quamoclit angulata*-derived novel compound (KRIBB-BH-P, 1 mg/kg), and the diabetic mouse group treated with the *Quamoclit angulata*-derived novel compound (KRIBB-BH-SFP, 1 mg/kg), in which the treated mice were orally administered with the compounds daily for 4 weeks, and the levels of glycated hemoglobin were measured using an ELISA kit (Cusabio biotech, Japan).

As a result, as shown in the following table 1, the level of glycated hemoglobin was significantly reduced in the diabetic mouse group treated with the *Quamoclit angulata-*derived novel compound (KRIBB-BH-SFP, 1 mg/kg) by oral administration as compared to the normal mouse control group.

**[Table 1]**

| | Initial Weight (g) | Final Weight (g) | HbA1c (%) |
|---|---|---|---|
| C57 | 22.2±1.36 | 24.5±1.32 | 5.81±0.19 |
| Control | 40.5±2.00 | 49.9±0.59 | 9.48±0.39 |
| KRIBB-BH-P | 39.1±1.66 | 49.9±0.38 | 6.63±0.70 |
| KRIBB-BH-SFP | 40.3±2.50 | 49.8±1.88 | 6.51±0.46 |

| | | | |
|---|---|---|---|
| KRIBB-BH-P: *Quamoclit* single-compound KRIBB-BH-SFP: *Quamoclit* single-compound fraction | | | |

### 6-3: Test of Blood Glucose

In order to conduct an oral glucose tolerance test,
5-week-old mouse (Male C57BL/6J, 19 g, Koatech, Pyeongtaek, Korea) was purchased and acclimated under constant temperature (25 °C) and humidity (50%) for 1 week and used for the experiment. Mice were classified into a group fed with normal diet and a group fed with high-fat diet and the mice were fed for 2 weeks, each group consisting of five mice.

### 6-3-1: Determination of Effect of Quamoclit angulata-Derived Compound on the lowering of blood glucose levels over time by fasting blood glucose test

At 2 weeks after feeding with high-fat diet, the experimental mice were fasted for 16 hours, and the fasting blood glucose was measured. Then, the compound was orally administered 1, 2, 4, 8, 16, and 24 hours before the measurement, followed by oral administration of 2 g/kg (body weight) of glucose, and then the level of blood glucose was measured was measured.

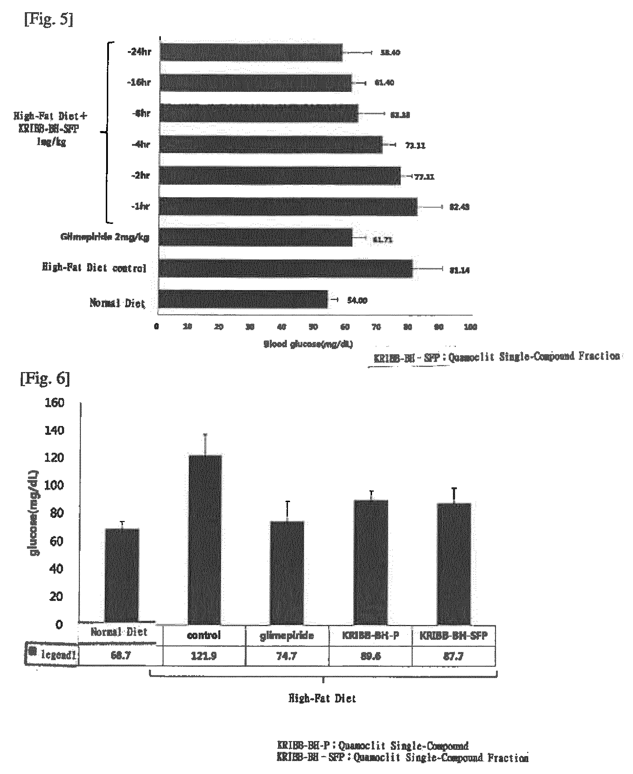

As a result, as shown in FIG. 5, the highest efficacy was shown 8 hours after the oral administration of the compound.

### 6-3-2: Determination of Effect of Quamoclit angulata-Derived Compound on the lowering of blood glucose levels by fasting blood glucose test

At 2 weeks after feeding with high-fat diet, the experimental mice were classified into a normal mouse control group, a diabetic mouse control group, a diabetic mouse group treated with drug (glimepiride), a diabetic mouse group treated with the *Quamoclit angulata*-derived novel compound (KRIBB-BH-P, 1 mg/kg), and a diabetic mouse group treated with the *Quamoclit angulata*-derived novel compound (KRIBB-BH-SFP, 1 mg/kg), and the treated mice were orally administered with the drug and compounds daily for 2 weeks. Then, the mice were fasted for 16 hours, and the level of blood glucose was measured.

As a result, with respect to the change in blood glucose, as shown in FIG. 6, the level of blood glucose was increased in the diabetic mouse control group as compared to the normal mouse control group; however, in the diabetic mouse group treated with the *Quamoclit angulata*-derived novel compound (KRIBB-BH-P, 1 mg/kg) and the diabetic mouse group treated with the *Quamoclit angulata*-derived novel compound (KRIBB-BH-SFP, 1 mg/kg), the level of blood glucose was significantly reduced.

While the present invention has been described in detail with reference to specific embodiments, it will be apparent to those skilled in the art that the detailed description is for illustration purposes only and is not intended to limit the scope of the present invention. Thus, the substantial scope of the present invention will be defined by the appended claims and equivalents thereof.

### [Industrial Applicability]

As described above, the novel composition derived from the plant of the genus *Quamoclit* and the composition comprising the same of the present invention have an excellent effect on the stimulation of insulin secretion, thus exhibiting efficacy in preventing or treating diabetes and various diabetic complications.

## Claims

1. A compound represented by the following chemical formula 1: 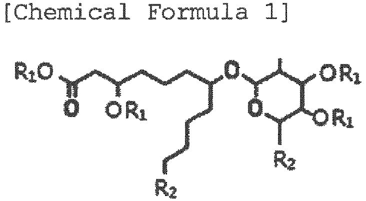 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

2. The compound of claim 1, wherein R₁ and R₂ are each independently H or C₁₋₂₀ alkyl.

3. The compound of claim 1, wherein both R₁ and R₂ are H.

4. The compound of claim 1, wherein the compound is derived from a plant of the genus *Quamoclit.*

5. The compound of claim 4, wherein the plant of the genus *Quamoclit* is *Quamoclit angulata.*

6. A method for preparing a compound represented by the following chemical formula 1, the method comprising the steps of:
(a) extracting a plant of the genus *Quamoclit* with a solvent to obtain a *Quamoclit* extract;
(b) fractionating the *Quamoclit* extract with a solvent to obtain a water fraction; and
(c) enzymatically or chemically treating the water fraction to obtain the compound represented by chemical formula 1: 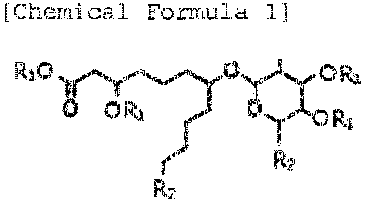
wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

7. A method for preparing a compound represented by the following chemical formula 1, the method comprising the steps of:
(a) extracting a plant of the genus *Quamoclit* with a solvent selected from the group consisting of water, an organic solvent, and a mixture thereof to obtain a *Quamoclit* extract;
(b) suspending the *Quamoclit* extract by addition of water, followed by sequential fractionation with hexane, ethyl acetate, and butanol to obtain a water fraction;
(c) isolating and purifying the water fraction to obtain a compound represented by the following chemical formula 3; and
(d) enzymatically or chemically treating the compound of chemical formula 3 to obtain the compound of chemical formula 1: 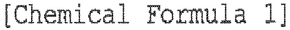 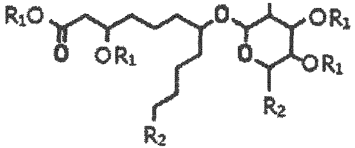 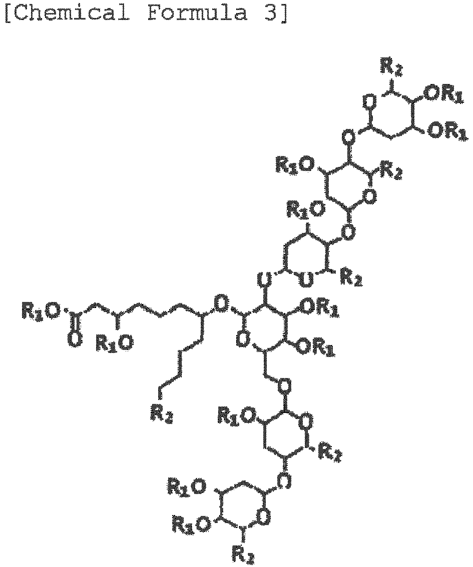
wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

8. A method for preparing a compound represented by the following chemical formula 1, the method comprising the step of enzymatically or chemically treating a compound represented by the following chemical formula 3 to obtain the compound represented by chemical formula 1: 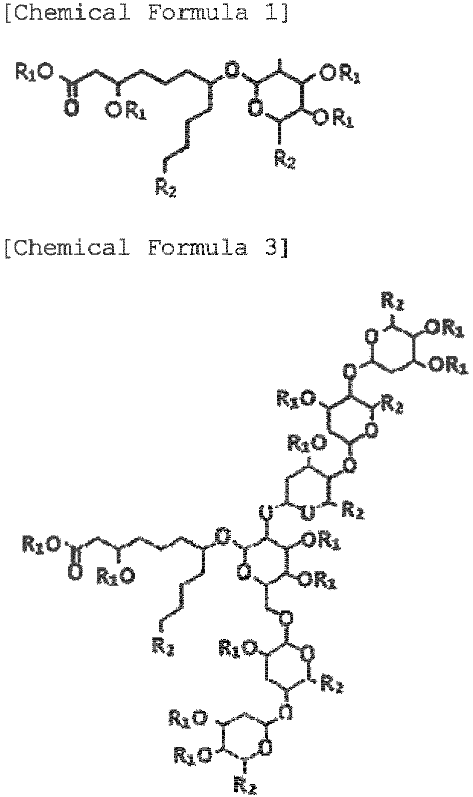 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

9. The method of claim 6 or 7, wherein the plant of the genus *Quamoclit* is *Quamoclit angulata.*

10. The method of any one of claims 6 to 8, wherein the enzymatic treatment comprises the steps of:
(i) treatment with at least one enzyme selected from the group consisting of cellulase, beta-glucanase, and xylanase; and
(ii) treatment with at least one enzyme selected from the group consisting of glucosidase, cellulase, galactosidase, amyloglucosidase, xylosidase, and xylanase.

11. A method for chemically synthesizing a compound represented by the following chemical formula 1, the method comprising the steps of:
(a) adding oxalyl chloride and dimethylsulfoxide to 1,5-hexenyl ether for reaction, adding triethylamine to the mixture for reaction, and then further adding alkylmagnesium bromide to the mixture for reaction in a reaction solvent;
(b) adding sugar, acetic anhydride, and iodine to the mixture for reaction, adding iodide to the mixture for reaction, and then further adding sodium hydrogen carbonate and sodium bisulfite to the mixture for reaction in a reaction solvent;
(c) adding a molecular sieve and zinc chloride to the product in step (a) and the product in step (b) for reaction in a reaction solvent;
(d) adding osmium tetroxide, sodium periodate, and tetrabutylammonium hydrogen sulfate to the product in step (c) for reaction in a reaction solvent;
(e) adding ethylacetate to the product in step (d) for reaction in the presence of lithium diisopropylamide in a reaction solvent;
(f) adding sodium alkoxide to the product in step (e) for reaction in a reaction solvent; and
(g) adding a base to the product in step (f) for reaction in a reaction solvent: 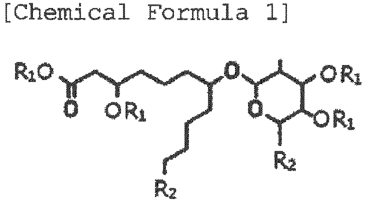
wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

12. The method of claim 11, wherein both R₁ and R₂ are H.

13. The method of claim 11, wherein the sugar in step (b) comprises at least one selected from the group consisting of glucose, fucose, rhamnose, arabinose, xylose, quinovose, maltose, glucuronic acid, ribose, N-acetyl glucosamine, and galactose.

14. The method of claim 11, wherein the reaction solvent comprises at least one selected from the group consisting of aliphatic or aromatic hydrocarbons, ketones, esters, halogenated hydrocarbons, ethers, alcohols, N,N-dimethylformamide, N,N-dimethylacetamide, and acetonitrile.

15. The method of claim 14, wherein the reaction solvent in steps (a) and (e) is tetrahydrofuran and the reaction solvent in steps (b) and (c) is dichloromethane.

16. The method of claim 14, wherein the reaction solvent in step (d) is a mixed solution of diethylether and water and the reaction solvent in steps (f) and (g) is anhydrous methanol.

17. The method of claim 11, wherein steps (a) and (e) are carried out at a temperature ranging from -60 to -80 °C and step (f) is carried out at a temperature ranging from -10 to 10 °C.

18. The method of claim 11, wherein the alkylmagnesium bromide in step (a) is pentylmagnesium bromide.

19. The method of claim 11, wherein the sodium alkoxide in step (f) is sodium methoxide.

20. A compound represented by the following chemical formula 6: 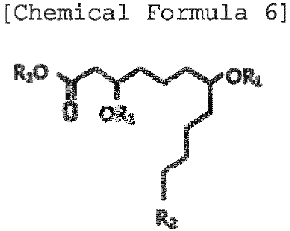 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

21. A method for chemically synthesizing a compound represented by the following chemical formula 6, the method comprising the steps of:
(a) adding oxalyl chloride and dimethylsulfoxide to 1,5-hexenyl ether for reaction, adding triethylamine to the mixture for reaction, and then further adding alkylmagnesium bromide to the mixture for reaction in a reaction solvent;
(b) adding acetic anhydride, pyridine, and 4-dimethylaminopyridine to the product in step (a) for reaction in a reaction solvent;
(c) adding osmium tetroxide, sodium periodate, and tetrabutylammonium hydrogen sulfate to the product in step (b) for reaction in a reaction solvent;
(d) adding ethylacetate to the product in step (c) for reaction in the presence of lithium diisopropylamide in a reaction solvent;
(e) adding sodium alkoxide to the product in step (d) for reaction in a reaction solvent; and
(f) adding a base to the product in step (e) for reaction in a reaction solvent: 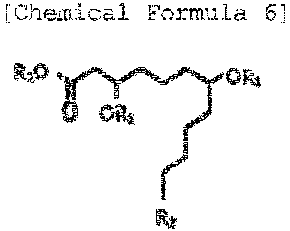
wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

22. The method of claim 21, wherein both R₁ and R₂ are H.

23. The method of claim 21, wherein the reaction solvent comprises at least one selected from the group consisting of aliphatic or aromatic hydrocarbons, ketones, esters, halogenated hydrocarbons, ethers, alcohols, N,N-dimethylformamide, N,N-dimethylacetamide, and acetonitrile.

24. The method of claim 23, wherein the reaction solvent in steps (a) and (d) is tetrahydrofuran and the reaction solvent in step (b) is dichloromethane.

25. The method of claim 23, wherein the reaction solvent in step (c) is a mixed solution of diethylether and water and the reaction solvent in steps (e) and (f) is anhydrous methanol.

26. The method of claim 21, wherein steps (a) and (d) are carried out at a temperature ranging from -60 to -80 °C and step (e) is carried out at a temperature ranging from 15 to 35 °C.

27. The method of claim 21, wherein the alkylmagnesium bromide in step (a) is pentylmagnesium bromide.

28. The method of claim 21, wherein the sodium alkoxide in step (e) is sodium methoxide.

29. A pharmaceutical composition for preventing or treating diabetes or diabetic complications, comprising a compound represented by the following chemical formula 1; a salt thereof; or an extract comprising the compound represented by chemical formula 1 or a salt thereof as an active ingredient: 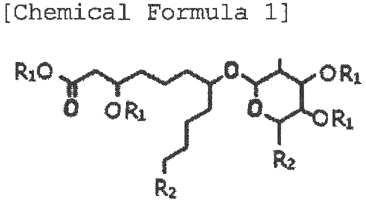 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

30. A pharmaceutical composition for preventing or treating diabetes or diabetic complications, comprising a compound represented by the following chemical formula 6; a salt thereof; or a compound comprising the compound represented by chemical formula 6 or a salt thereof as an active ingredient: 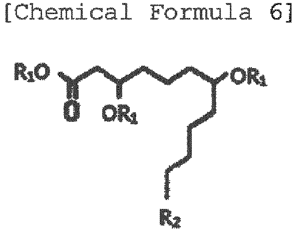 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

31. The pharmaceutical composition of claim 29 or 30, wherein R₁ and R₂ are each independently H or C₁₋₂₀ alkyl.

32. The pharmaceutical composition of claim 29 or 30, further comprising an extract obtained by plant-culture or tissue-culture of *Quamoclit* and a compound obtained from the extract.

33. The pharmaceutical composition of claim 32, wherein both R₁ and R₂ are H.

34. The pharmaceutical composition of claim 29 or 30, wherein the diabetic complications are selected from the group consisting of hyperglycemia, hyperinsulinemia, hypertriglyceridemia, insulin resistance, dyslipidemia, impaired fasting glucose, impaired glucose tolerance, obesity, arteriosclerosis, microangiopathy, renal disease, heart disease, foot ulcer, arthritis, osteoporosis, and ophthalmologic disease induced by diabetes.

35. The pharmaceutical composition of claim 34, wherein the ophthalmologic disease induced by diabetes is selected from the group consisting of diabetic retinopathy, cataract, macular degeneration, external ophthalmoplegia, iridocyclitis, optic neuritis, glaucoma, retinal degeneration, fundus hemorrhage, anomalies of refraction, subconjunctival hemorrhage, and vitreous hemorrhage.

36. The pharmaceutical composition of claim 29 or 30, further comprising an antidiabetic compound.

37. The pharmaceutical composition of claim 36, wherein the antidiabetic compound comprises at least one selected from the group consisting of nateglinide, repaglinide, glitazones, sulfonylureas, metformin, glimepiride, thiazolidinediones, biguanides, α-glucosidase inhibitor such as acarbose, and meglitinides such as prandin.

38. A health functional food for preventing diabetes and diabetic complications, comprising a compound represented by the following chemical formula 1; a salt thereof; or an extract comprising the compound represented by chemical formula 1 or a salt thereof as an active ingredient: 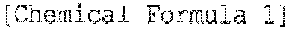 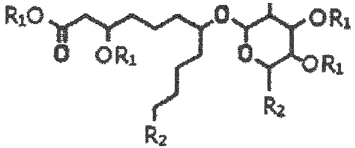 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

39. A health functional food for preventing diabetes and diabetic complications, comprising a compound represented by the following chemical formula 6; a salt thereof; or a compound comprising the compound represented by chemical formula 6 or a salt thereof as an active ingredient: 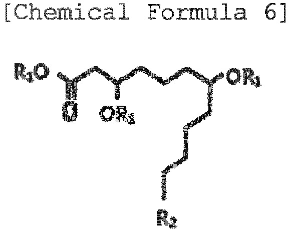 wherein R₁ and R₂ are each independently hydrogen, C₁₋₂₀ alkyl, C₆₋₃₀ aryl, C₂₋₂₀ allyl, or C₆₋₃₀ arylalkyl or acyl, and wherein the alkyl and aryl are unsubstituted or substituted with a substituent selected from the group consisting of vinyl, phenyl, halogen, nitro, amino, acryl, epoxy, amide, C₁₋₇ alkoxy, C₁₋₇ haloalkoxy, and hydroxy.

40. The health functional food of claim 38 or 39, wherein R₁ and R₂ are each independently H or C₁₋₂₀ alkyl.

41. The health functional food of claim 38 or 39, further comprising an extract obtained by plant-culture or tissue-culture of *Quamoclit* and a compound obtained from the extract.

42. The health functional food of claim 40, wherein both R₁ and R₂ are H.

43. The health functional food of claim 38 or 39, wherein the diabetic complications are selected from the group consisting of hyperglycemia, hyperinsulinemia, hypertriglyceridemia, insulin resistance, dyslipidemia, impaired fasting glucose, impaired glucose tolerance, obesity, arteriosclerosis, microangiopathy, renal disease, heart disease, foot ulcer, arthritis, osteoporosis, and ophthalmologic disease induced by diabetes.

44. The health functional food of claim 43, wherein the ophthalmologic disease induced by diabetes is selected from the group consisting of diabetic retinopathy, cataract, macular degeneration, external ophthalmoplegia, iridocyclitis, optic neuritis, glaucoma, retinal degeneration, fundus hemorrhage, anomalies of refraction, subconjunctival hemorrhage, and vitreous hemorrhage.
